# EUROPEAN PATENT SPECIFICATION

(11) **EP 1 279 665 B1**
(45) Date of publication and mention of the grant of the patent: **20.02.2008**
(21) Application number: 02016262.4
(22) Date of filing: 23.07.2002
(51) Int. Cl.: C07D 209/42, C07D 263/44

(54) **A process for the preparation of perindopril, its analogous compounds and salts thereof using 2,5-dioxo-oxazolidine intermediate compounds**
Ein Verfahren zur Herstellung von Perindopril, analogen Verbindungen dazu sowie Salzen davon unter Verwendung von 2,5-Dioxooxazolidin-Zwischenprodukten
Un procédé pour la préparation de périndopril, leurs composés analogues et leurs sels en utilisant 2,5-dioxo-oxazolidines comme intermédiaires

(30) Priority: 24.07.2001 EP 01500197
(43) Date of publication of application: 29.01.2003
(73) Proprietor: ADIR, F-92415 Courbevoie Cedex (FR)
(72) Inventor: Cid, Pau, Barcelona 08080 (ES)
(74) Representative: Campbell, Neil Boyd

(56) References cited:
- EP-A1- 0 308 341
- EP-A1- 0 309 324
- WO-A1-01/56986
- US-A- 4 686 295
- US-A- 5 359 086
- DATABASE CA [Online] CHEMICAL ABSTRACTS SERVICE, COLUMBUS, OHIO, US; SKILES, JERRY W. ET AL: "Syntheses of N-(carboxymethyl) dipeptides via N-carboxy.alpha.-amino acid anhydride (NCA) intermediates" retrieved from STN Database accession no. 109:129642 XP002216969 & ORG. PREP. PROCED. INT. (1988), 20(1-2), 109-15,

## Description

This invention relates to a new process for the preparation of perindopril (2S,3aS,7aS)-1-[(S)-N-[(S)-1-carboxybutyl]alanyl]hexahydro-2-indoline carboxylic acid 1-ethylester and analogues and salts thereof, especially tert-butylamine salts.

Perindopril and its tert-butylamine salt perindopril erbumine are used as inhibitors of angiotensin-converting enzyme (ACE).

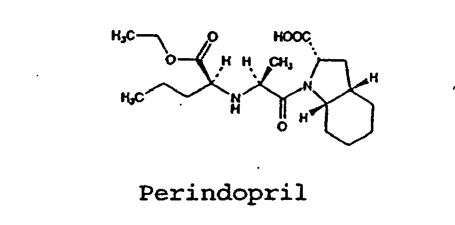

Perindopril acts as a prodrug of the diacid perindoprilat, its active form. Following oral administration perindopril is rapidly absorbed and extensively metabolised, mainly in the liver, to perindoprilat and inactive metabolites including glucuronides.

Perindopril is used in the treatment of hypertension and heart failure since angiotension-converting enzyme inhibitors inhibit the conversion of angiotension I to angiotensin II. They are antihypertensive agents that act as vasodilators and reduce peripheral resistance; they have beneficial effects on left ventricular dysfunction and they reduce protein urea associated with kidney disease.

Other areas of potential therapy are in myocardial infarction and diabetic nephropathy although adverse affects including hypotension, skin rashes, angioedema, cough, taste disturbances, impairment of renal function and hyperkalaemia have been reported.

Perindopril was first synthesised by a process described in EP-A-0049658. Today, perindopril is conventionally prepared by the processes described in detail below.

The first process is a four stage process starting from a perhydroindole carboxylic acid which must first be protected before reaction takes place. The N side chain is then prepared as shown in Scheme 1 by coupling a suitably protected perhydroindole carboxylic acid with a reactive derivative of an enantiomerically pure amino acid such as alanine. The remainder of the side chain is formed by reductive amination, conventionally achieved using a metal hydride such as sodium cyanoborohydride. Deprotection is then effected.

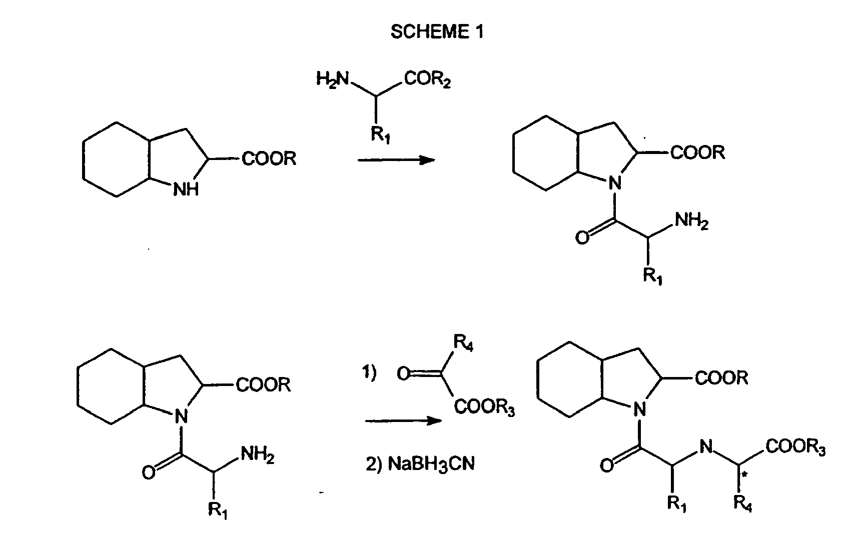

Including both the carboxy protection and deprotection steps, this synthetic route to perindopril comprises four steps and the reductive amination stage leads to the formation of two possible stereoisomers that have to be separated. In order to produce an enantomerically pure drug therefore, a difficult separation procedure needs to be conducted once the actual perindopril has been prepared.

An alternative method for preparing perindopril is described in Scheme 2 and this involves the coupling of a preformed side chain with the suitably protected perhydroindole carboxylic acid species, such as dicyclohexylcarbodiimide (DCC). Again including both the protection and deprotection steps, this method requires three steps.

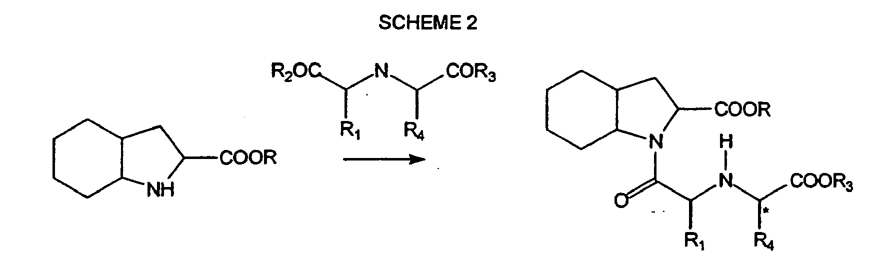

It should also be noted that in both the above syntheses the final step involves the deprotection of the carboxylate group attached to the perhydroindole, normally performed by catalytic hydrogenation (e.g. where the protecting group is a benzyl species) or in acid conditions (e.g. where the protecting group is a tert-butyl species). Moreover, the deprotection step may cause epimerisation of some of the stereocentres in the molecule.

The inventors have devised a new process for the preparation of perindopril and analogues and salts thereof which involves only two simple stages and does not require the problematic use of protecting groups. Moreover, the synthesis gives rise to enantiomerically pure products without the need for any stereoisomer separation processes. The process involves the use of an oxazolidine species which is subsequently opened to form perindopril or analogues thereof.

The only byproduct in this coupling reaction is CO₂ and the process avoids the use of coupling agents such as DCC and the corresponding formation of problematic byproducts such as dicyclohexylurea which is notoriously difficult to remove from a reaction mixture.

Thus, viewed from one aspect the invention provides a process for the preparation of a compound of formula (IV) 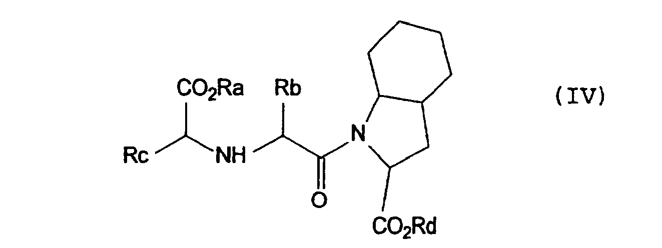 or an ester or a salt thereof comprising:
1) reacting a compound of formula (I) 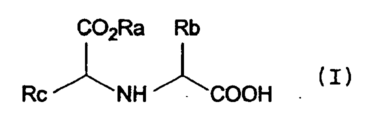 (wherein Rₐ represents C₁₋₄ alkyl, R_{b} represents C₁₋₄ alkyl and R_{c} represents propyl) with a compound of formula X₂C=O (wherein each X independently represents a leaving group) to give a compound of formula (II) 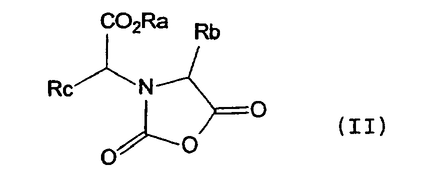 (wherein Rₐ, R_{b} and R_{c} are as hereinbefore defined) ; and
2) reacting said compound of formula (II) with a compound of formula (III) 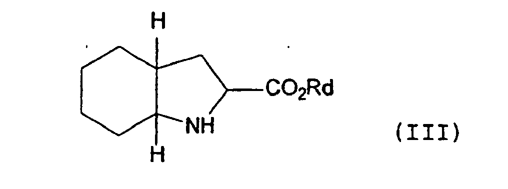 (wherein R_{d} represents hydrogen or a protecting group).

In the compound of formula (I), Rₐ is preferably methyl or especially ethyl. R_{b} is preferably ethyl or especially methyl.

It is also preferred if the stereochemistry of the two stereocentres in the compounds of formula (I) are (S). The compound of formula (I) is therefore most preferably a compound of formula (A) 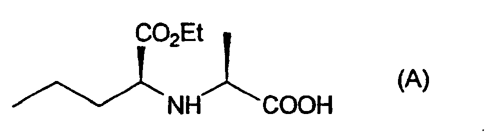 Compounds of formula (I) may be prepared by techniques known in the art. For example, a compound of formula (A) can be prepared from the reaction of an optionally protected alanine with a suitably functionalised pentanoic acid ester.

Alternatively, compounds of formula (I) may be prepared as shown in the reaction scheme below: 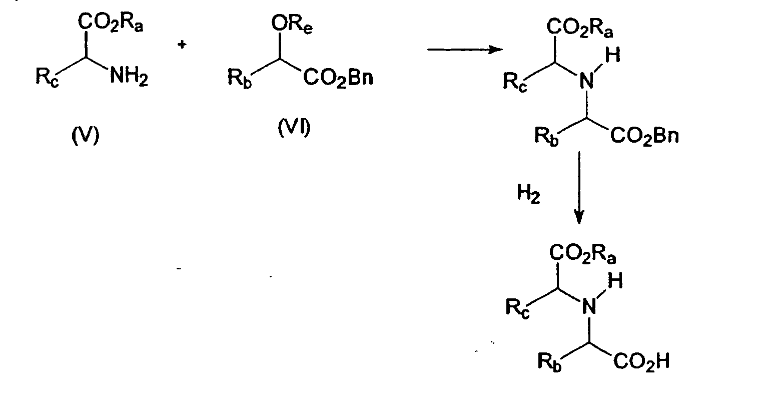 wherein Rₐ, R_{b} and R_{c} are as hereinbefore defined and Rₑ, together with the oxygen atom to which it is attached forrms a leaving group, e.g. -OSO₂CF₃. Preferences for Rₐ and R_{b} are as hereinbefore described. The compound of formula (VI) can be prepared from D-lactic acid by conventional processes. The stereochemistry of the compound of formula (V) is preferably (S) to allow the preparation of a compound of formula (A). Deprotection of the carboxyl group is accomplished by hydrogenation.

The compound of formula (I) is reacted with a compound capable of introducing a carbonyl group so as to allow the formation of the oxazolidine. Use of oxazolidine species is disclosed, for example, in US 4,686,295 and US 5,359,086. A comprehensive discussion of the synthesis of oxazolidines (also referred to as amino acid-N-carboxy anhydrides) can be found in the book α-Aminoacid-N-carboxy anhydrides and related heterocycles, syntheses, properties, peptide synthesis, polymerisation by Hans Rytger Kricheldorf (Springer-Verlag, Berlin 1987) which is herein incorporated by reference. Thus, the oxazolidine ring may be formed by the Fuchs-Farthing method as described therein.

The Fuchs-Farthing method involves the direct reaction of free amino acids with phosgene, the reaction proceeding via an N-chloro-formyl amino acid intermediate which is converted to the anhydride in the presence of hydrochloric acid.

Suitable compounds capable of introducing a carbonyl group are of formula X₂C=O. Each X may independently be any suitable leaving group which are well known in the art. Thus, each X must be capable of being displaced by the nucleophilic lone pairs present on the oxygen and nitrogen atoms of compound (I).

For example, X may be a halogen, tosylate, mesylate, alkoxy group, alkylthio or imidazolyl group. In general where X forms an ester or thioester linkage with the CO moiety, a compound suitable for introducing a carbonyl will result e.g. (Cl₃CO)-. In a preferred embodiment both X's are the same and in a still yet further preferred embodiment both X's represent halogen, preferably chlorine. In this instance, the compound of formula X₂C=O is of course phosgene (Cl₂C=O).

Since phosgene is hazardous to handle, it may be preferable to use it in its less active form, triphosgene ((CCl₃O)₂CO.

In another preferred embodiment, X₂C=O is N,N'-carbonyldiimidazole.

The nucleophilic nitrogen and oxygen atoms on the compound of formula (I) attack the electrophilic X₂C=O species allowing a 5-endo-trig cyclisation to occur. Depending on the nature of the X₂CO group, this reaction can be carried out in a variety of solvents. In particular, most inert, low boiling solvents are useful as reaction media, e.g. tetrahydrofuran, dioxane, dichloromethane.

When the carbonyl introducing agent is triphosgene or phosgene the skilled chemist will appreciate that careful control over the reaction is required in order to avoid potential hazardous conditions.

For example, the reaction may be carried out in a water/ dichloromethane mixture in the presence of sodium monohydrogen phosphate.

Before isolation of the compound of formula (II), it may be necessary to neutralise any residual carbonyl introducing agent, e.g. by addition of a base such as pyridine. The compound of formula (II) may then be isolated using standard work-up techniques and washing phases.

The conversion through to compound (II) can be achieved in yields in excess of 70%, e.g. 80% without any loss of stereochemistry. In a preferred embodiment, the compound of formula (II) is of formula (B)

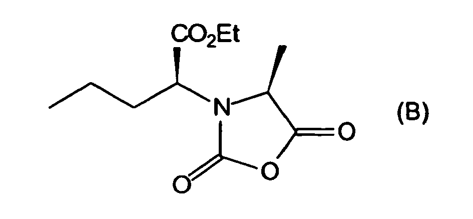

Some compounds of formula (II), especially the compound of formula (B) are new. Compounds of formula (II) wherein Rc is propyl form a further aspect of the invention. Hence, viewed from a further aspect the invention provides a compound of formula (II) as described in the claims.

Compound (II) is then contacted with a compound of formula (III). In the compounds of formula (III), Rd is preferably a hydrogen atom, however it may represent a protecting group such as benzyl. Compounds of formula (III) have been widely described in the literature and their preparation is described, *inter alia,* in EP-A-0037231. This reaction may take place in a suitable organic solvent, e.g. dichloromethane in the presence of a weak base, e.g. triethylamine. The preferred compound of formula (III) is of formula (C): 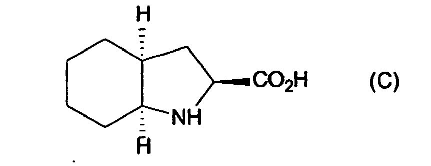 i.e. (2S, 3aS, 7aS)-2-carboxyperhydroindole.

The product of the reaction of compounds of formulae (B) and (C) is, of course, perindopril which can be purified by conventional techniques or crystallised immediately as a salt, e.g. a tert-butyl amine salt.

After the reaction of compounds (II) and (III), and if necessary deprotection of the perhydroindolecarboxylic acid, isolation of the product may take place. In this regard, water may be added to the reaction mixture and the mixture cooled to 15°C. The pH of the mixture may be adjusted to approximately 4.2 by the addition of acid, e.g. hydrochloric acid, and the aqueous phase extracted with dichloromethane. The organic extract may then be dried under reduced pressure below 40°C to yield an oil.

The reaction of compounds (II) and (III) can be performed without isolation of the compound of formula (II) from the original medium.

This can be converted to the tertbutylamine salt (e.g. perindopril erbumine) simply by contacting the oil in an appropriate solvent with tertbutylamine. After isolation, the salt may be isolated in excess of 70% yield.

The perindopril or derivative thereof produced by the process of the invention can be used in the indications discussed in the background section of the text and in indications known to the skilled person. The perindopril or derivatives thereof may be formulated as part of a pharmaceutical composition and administered by any standard routes such as oral, transmucosal or by injection.

The invention will now be described further in relation to the following non-limiting Examples.

### Example 1

### 2,5-dioxo-3-[1-(S)-ethoxycarbonyl-butyl]-4-(S)-methyl-oxazolidine

A solution of 28.7 g of sodium monohydrogen phosphate in 200 mL of water was prepared and warmed to 30-35°C. After complete dissolution, the mixture was cooled to room temperature and charged with 160 mL of dichloromethane. 20 g of N-[1-(S)-ethoxycarbonylbutyl]-(S)-alanine was added over the well stirred mixture and the resulting mixture cooled to 15 °C. A solution of 10.9 g of triphosgene in 40 mL of dichloromethane was slowly added over 30 min keeping the temperature below 20 °C.

After triphosgene addition, the mixture was stirred for 30 min and 0.1 mL of pyridine added to destroy residual phosgene. After stirring for a further 1 hour, or until the phosgene is completely destroyed, the phases were separated and the organic phase washed first with 100 mL HCl 2N and then with 100 mL of water. The organic phase was filtered and the solvent evaporated at reduced pressure.
18.85 g of a pale yellow oil was obtained.
Assay: 95% (of 2,5-dioxo-3-[1-(S)-ethoxycarbonylbutyl]-4-(S)-methyl-oxazolidine).
Yield: 80%

### Example 2

### tert-butylamine (2S,3aS,7aS)-1-{2-[1-ethoxycarbonyl)-(S)-butylaminol-(S)-propionyl}-octahydroindol-2-carboxylate (PERINDOPRIL ERBUMINE)

20 g of (2S,3aS,7aS)-2-carboxyoctahydroindole was suspended in 150 mL of dichloromethane at 25°C and 16.5 mL of triethylamine added. A solution of 27.5 g of 95% 2,5-dioxo-3-[1-(S)-ethoxycarbonyl-butyl]-4-(S)-methyl-oxazolidine (Example 1) in 40 mL of dichloromethane was slowly added over 3 hours and the mixture stirred for a further 1 hour.

150 mL of water was added and the biphasic mixture cooled to 15°C. The pH was adjusted to 4.2 by the addition 2N hydrochloric acid (=52 mL was needed). The organic phase was separated and the aqueous phase extracted with 100 mL of dichloromethane. The organic extracts were combined and filtered and the solvent evaporated at reduced pressure keeping the temperature below 40 °C to obtain an oil. To the oil was added 100 mL of acetonitrile and the solvent then removed *in vacuo.*

The resulting oil was dissolved in 300 mL of acetonitrile and the solution warmed to 35 °C. A solution of 12.5 mL of tert-butylamine in 50 ml of acetonitrile was added slowly over 30 mins and the resulting mixture stirred at 40 °C for 1 hour. The mixture was cooled and stirred at 5 °C for a further 1 hour. The resulting precipitate was filtered and washed with 50 ml of acetonitrile twice.

78.27g of wet white solid are obtained (42.34 g of dry product calculated by loss on drying).

70.27 g of wet solid was suspended in 160 mL of acetonitrile and 4.75 mL of water added. The mixture was stirred at 40 °C for 1 hour before being cooled and stirred at 5 °C for 1 hour. The solid was filtered and washed with 50 ml of acetonitrile twice.

After drying, 44.52 g of perindopril erbumine are obtained as a white powder.
Yield: 81%

### Example 3

### Tert-butylamine (2S, 3aS, 7aS)-1-{2-[(1-ethoxycarbonyl)-(S)-butylaminol-(S)-propionyl}-octahydroindol-2-carboxylate (Perindopril Erbumine)

To a cooled suspension of 5g of N-{1-(S)ethoxycarbonylbutyl}-(S)-alanine in 71 ml of dichloromethane, 4.47 g of N,N'-carbonyldiimidazole was added. The mixture was cooled at 0°C and stirred for 1 hour.

5.05 g of (2S, 3aS, 7aS)-2-carboxyoctahydroindole was added at -5°C to the above solution and the mixture stirred at -5°C for 1 hour. The dichloromethane was evaporated and 71 ml water was added to the aqueous phase followed by addition of 11.6 ml of 6N hydrochloric acid.

The aqueous solution was saturated with sodium chloride and extracted with 120 ml of dichloromethane. The solvent was evaporated.

The resulting oil was dissolved in 80 ml of ethylacetate. 1.78 g of tert-butylamine was added whilst stirring at room temperature. The mixture was warmed to aid dissolution then cooled to 20°C. The resulting precipitate was filtered and washed with ethyl acetate.
Yield 80%, purity <99%.

## Claims

1. A process for the preparation of a compound of formula (IV) 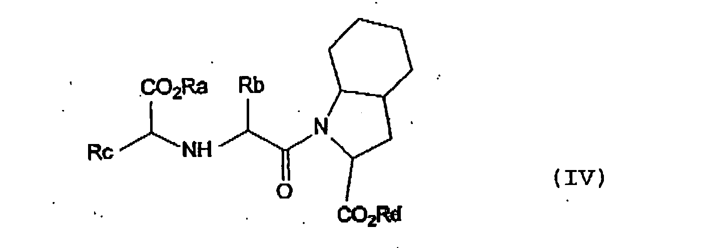 or an ester or a salt thereof comprising
1) reacting a compound of formula (I) 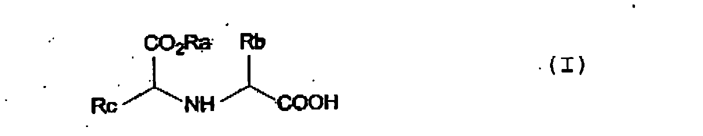 (wherein Rₐ represents C₁₋₄ alkyl, R_{b} represents C₁₋₄ alkyl and R_{c} represents propyl) with a compound of formula X₂C=O (wherein each X independently represents a leaving group) to give a compound of formula (II) 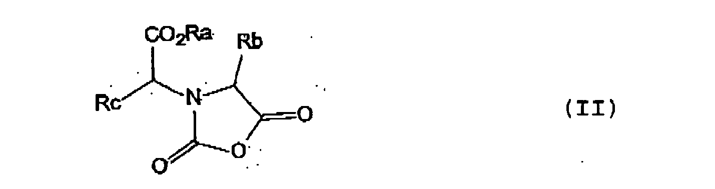 (wherein Rₐ, R_{b} and R_{c} are as hereinbefore defined); and
2) reacting said compound of formula (II) with a compound of formula (III) 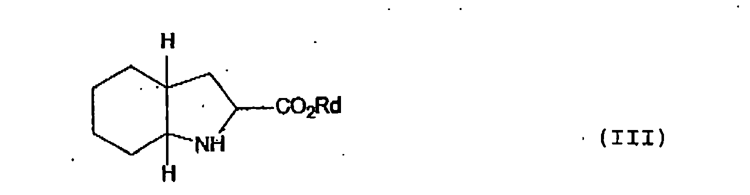 (wherein R_{d} represents hydrogen or a protecting group).

2. A process as claimed in claim 1 wherein Rd is hydrogen.

3. A process as claimed in claim 1 or 2 wherein Ra is ethyl.

4. A process as claimed in any one of claims 1 to 3 wherein Rb is methyl.

5. A process as claimed in any one of claims 1 to 4 comprising
1) reacting a compound of formula (A) 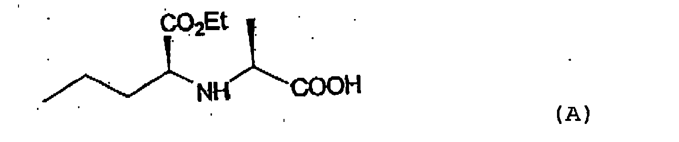 with a compound of formula X₂C=O (wherein each X independently represents a leaving group) to give a compound of formula (B) 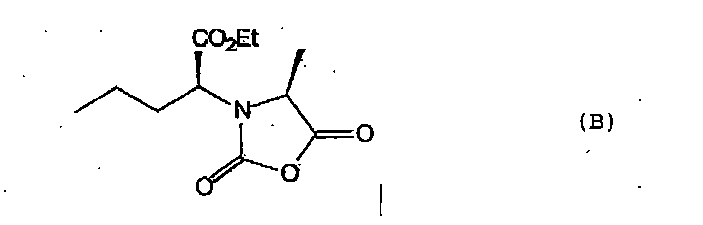
2) reacting said compound of formula (B) with the compound of formula (C)

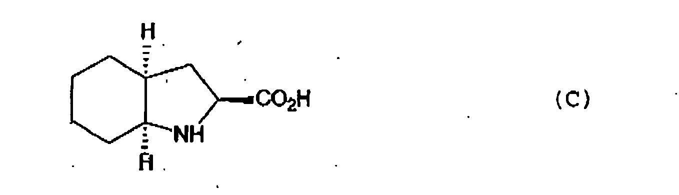

6. A process as claimed in claim 5 further comprising reacting a compound of formula (IV) with tertbutylamine.

7. A compound of formula (II) 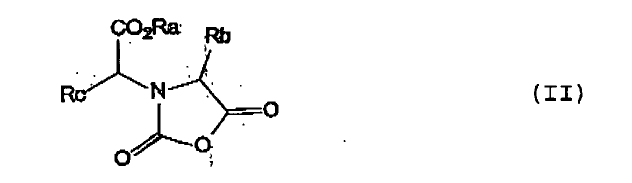 (wherein Rₐ represents C₁₋₄ alkyl, R_{b} represents C₁₋₄ alkyl and R_{c} represents propyl).

## Patentansprüche

1. Verfahren zur Herstellung einer Verbindung der Formel (IV) 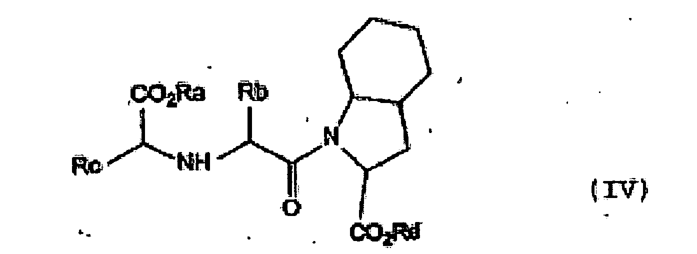 oder eines Esters oder eines Salzes davon, umfassend
1) Umsetzen einer Verbindung der Formel (I) 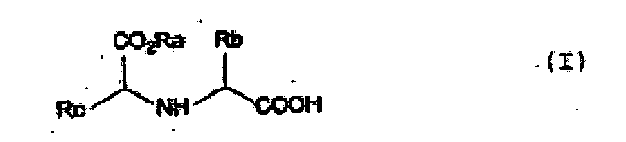 (worin Rₐ die Bedeutung C₁₋₄-Alkyl hat, R_{b} die Bedeutung C₁₋₄-Alkyl hat und R_{c} die Bedeutung Propyl hat)
mit einer Verbindung der Formel X₂C=O (worin jedes X unabhängig voneinander eine Abgangsgruppe bedeutet), um eine Verbindung der Formel (II) zu erhalten 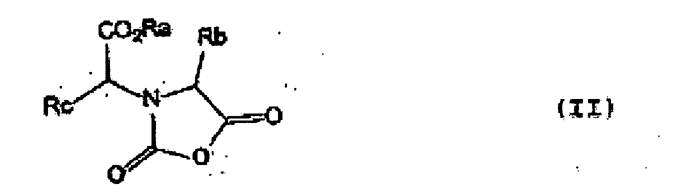 (worin Rₐ, R_{b} und R_{c} wie oben definiert sind); und
2) Umsetzen der Verbindung der Formel (II) mit einer Verbindung der Formel (III) 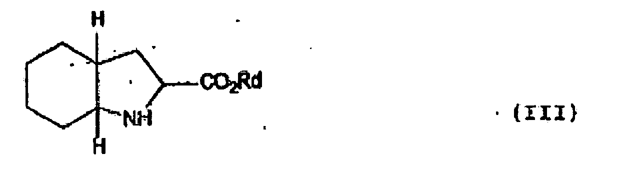 (worin R_{d} Wasserstoff oder eine Schutzgruppe bedeutet).

2. Verfahren nach Anspruch 1, worin R_{d} Wasserstoff ist.

3. Verfahren nach Anspruch 1 oder 2, worin Rₐ Ethyl ist.

4. Verfahren nach mindestens einem der Ansprüche 1 bis 3, worin R_{b} Methyl ist.

5. Verfahren nach mindestens einem der Ansprüche 1 bis 4, umfassend
1) Umsetzen einer Verbindung der Formel (A) 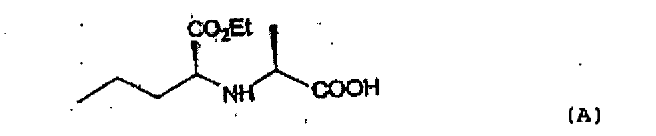 mit einer Verbindung der Formel X₂C=O (worin jedes X unabhängig eine Abgangsgruppe bedeutet), um eine Verbindung der Formel (B) zu erhalten 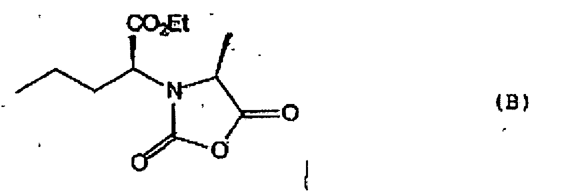
2) Umsetzen der Verbindung der Formel (B) mit der Verbindung der Formel (C)

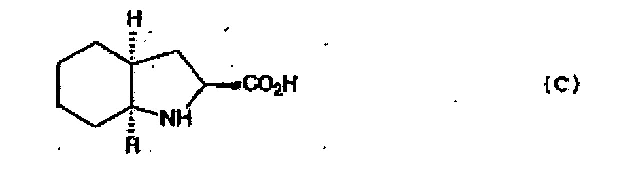

6. Verfahren nach Anspruch 5, umfassend weiterhin das Umsetzen einer Verbindung der Formel (IV) mit tert-Butylamin.

7. Verbindung der Formel (II) 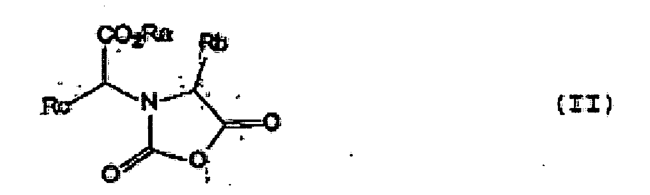 (worin Rₐ die Bedeutung C₁₋₄-Alkyl hat, R_{b} die Bedeutung C₁₋₄-Alkyl hat und R_{c} die Bedeutung Propyl hat).

## Revendications

1. Procédé de préparation d'un composé de formule (IV) : 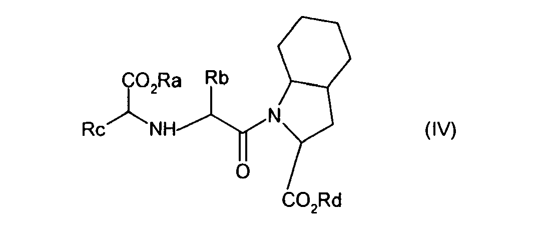 ou d'un ester ou d'un sel d'un tel composé, lequel procédé comporte :
1) le fait de faire réagir un composé de formule (I) : 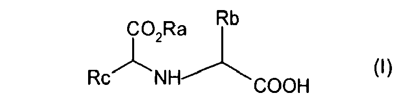 dans laquelle Rₐ représente un groupe alkyle en C₁₋₄, R_{b} représente un groupe alkyle en C₁₋₄, et R_{c} représente un groupe propyle, avec un composé de formule X₂C=O où chaque symbole X représente indépendamment un groupe partant, de manière à obtenir un composé de formule (II) : 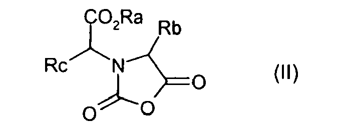 dans laquelle Rₐ, R_{b} et R_{c} ont les significations indiquées ci-dessus ;
2) et le fait de faire réagir ledit composé de formule (II) avec un composé de formule (III): 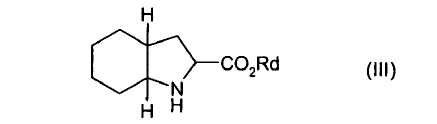
dans laquelle R_{d} représente un atome d'hydrogène ou un groupe protecteur.

2. Procédé conforme à la revendication 1, dans lequel R_{d} représente un atome d'hydrogène.

3. Procédé conforme à la revendication 1 ou 2, dans lequel Rₐ représente un groupe éthyle.

4. Procédé conforme à l'une des revendications 1 à 3, dans lequel R_{b} représente un groupe méthyle.

5. Procédé conforme à l'une des revendications 1 à 4, qui comporte les étapes suivantes :
1) le fait de faire réagir un composé de formule (A) : 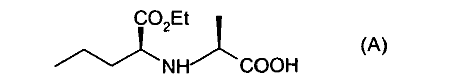 avec un composé de formule X₂C=O où chaque symbole X représente indépendamment un groupe partant, de manière à obtenir un composé de formule (B) : 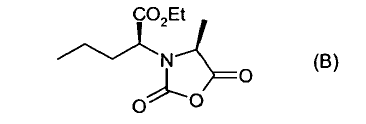
2) et le fait de faire réagir ledit composé de formule (B) avec un composé de formule (C) :

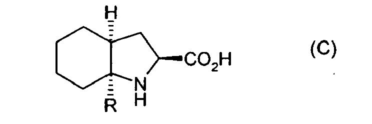

6. Procédé conforme à la revendication 5, qui comporte en outre le fait de faire réagir un composé de formule (IV) avec de la tertiobutylamine.

7. Composé de formule (II) : 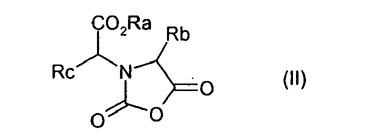 dans laquelle Rₐ représente un groupe alkyle en C₁₋₄, R_{b} représente un groupe alkyle en C₁₋₄, et R_{c} représente un groupe propyle.
